(19)

**Europäisches Patentamt**
**European Patent Office**
**Office européen des brevets**

(11) **EP 4 285 367 B1**

(12) **EUROPEAN PATENT SPECIFICATION**

(45) Date of publication and mention
of the grant of the patent:
**04.06.2025 Bulletin 2025/23**

(21) Application number: **22746262.9**

(22) Date of filing: **27.01.2022**

(51) International Patent Classification (IPC):
**G16B 5/00** *(2019.01)*       **G16B 40/20** *(2019.01)*
**G16B 45/00** *(2019.01)*

(52) Cooperative Patent Classification (CPC):
**G16B 5/20; G16B 40/00**

(86) International application number:
**PCT/KR2022/001512**

(87) International publication number:
**WO 2022/164236 (04.08.2022 Gazette 2022/31)**

(54) **METHOD AND SYSTEM FOR SEARCHING TARGET NODE RELATED TO QUERIED ENTITY IN NETWORK**

VERFAHREN UND SYSTEM ZUM SUCHEN EINES ZIELKNOTENS IN BEZUG AUF EINE ABGEFRAGTE EINHEIT IN EINEM NETZWERK

PROCÉDÉ ET SYSTÈME DE RECHERCHE DE NOEUD CIBLE ASSOCIÉ À UNE ENTITÉ INTERROGÉE DANS UN RÉSEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.01.2021 KR 20210012015
03.05.2021 KR 20210057257**

(43) Date of publication of application:
**06.12.2023 Bulletin 2023/49**

(73) Proprietor: **Standigm Inc.
Seoul 06261 (KR)**

(72) Inventors:
• **HAN, Seokjin**
  **Seoul 06261 (KR)**
• **KIM, Tae Yong**
  **Seoul 06261 (KR)**
• **KOO, Hee Jung**
  **Seoul 06261 (KR)**
• **YUN, So Jeong**
  **Seoul 06261 (KR)**

(74) Representative: **Frenkel, Matthias Alexander
Wuesthoff & Wuesthoff
Patentanwälte und Rechtsanwalt PartG mbB
Schweigerstraße 2
81541 München (DE)**

(56) References cited:
**KR-A- 20190 125 840      KR-A- 20200 079 608
KR-B1- 101 450 784      KR-B1- 102 288 957**

• **BO WANG ET AL: "Network Enhancement: a general method to denoise weighted biological networks", ARXIV.ORG, CORNELL UNIVERSITY LIBRARY, 201 OLIN LIBRARY CORNELL UNIVERSITY ITHACA, NY 14853, 9 May 2018 (2018-05-09), XP081141667, DOI: 10.1038/S41467-018-05469-X**
• **ZEN YE: "Curvature graph network", INTERNATIONAL CONFERENCE ON LEARNING REPRESENTATIONS, 31 December 2020 (2020-12-31), XP093165202, Retrieved from the Internet <URL:https://arxiv.org/pdf/2106.15762> DOI: 10.1609/aaai.v36i4.20333**

EP 4 285 367 B1

- MARZIEH EIDI: "Edge-based analysis of networks: curvatures of graphs and hypergraphs", THEORY IN BIOSCIENCES, vol. 139, no. 4, 20 November 2020 (2020-11-20), Berlin/Heidelberg, pages 337 - 348, XP093165210, ISSN: 1431-7613, Retrieved from the Internet <URL:http://link.springer.com/article/10.1007/s12064-020-00328-0/fulltext.html> DOI: 10.1007/s12064-020-00328-0
- SEOKJIN HAN ET AL: "PWN: enhanced random walk on a warped network for disease target prioritization", BMC BIOINFORMATICS, BIOMED CENTRAL LTD, LONDON, UK, vol. 24, no. 1, 21 March 2023 (2023-03-21), pages 1 - 21, XP021316163, DOI: 10.1186/S12859-023-05227-X
- BO WANG; ARMIN POURSHAFEIE; MARINKA ZITNIK; JUNJIE ZHU; CARLOS D. BUSTAMANTE; SERAFIM BATZOGLOU; JURE LESKOVEC: "Network Enhancement: a general method to denoise weighted biological networks", ARXIV.ORG, CORNELL UNIVERSITY LIBRARY, 201 OLIN LIBRARY CORNELL UNIVERSITY ITHACA, NY 14853, 9 May 2018 (2018-05-09), 201 Olin Library Cornell University Ithaca, NY 14853 , XP081141667, DOI: 10.1038/s41467-018-05469-x
- EIDI MARZIEH; FARZAM AMIRHOSSEIN; LEAL WILMER; SAMAL AREEJIT; JOST JÜRGEN: "Edge-based analysis of networks: curvatures of graphs and hypergraphs", THEORY IN BIOSCIENCES, ELSEVIER, BERLIN/HEIDELBERG, vol. 139, no. 4, 1 January 1900 (1900-01-01), Berlin/Heidelberg , pages 337 - 348, XP037312242, ISSN: 1431-7613, DOI: 10.1007/s12064-020-00328-0

## Description

## Technical Field

The present disclosure relates to a method for searching for a target node related to a queried entity in a network including a plurality of nodes and edges, and more specifically to a method and a system, whereby weights are assigned to the plurality of edges so that signal propagation can be made centered on the edges having high weights and thus a target node can be searched for with high accuracy.

## Background Art

Searching for target genes or proteins for treating a disease is a first step in drug development and is an important task that has a decisive influence on the success rate of drug development. Recently, it has become possible to process data with high throughput due to technology development. However, processing efficiency and accuracy may be lowered because conventional random walk-based methods rely on an undirected network to diffuse the information assigned to nodes.

Korean Patent Laid-open Publication No. 10-2016-0149623 discloses an apparatus for predicting pharmacodynamic drug-drug interactions through signal propagation interference in a protein-protein interaction network. It uses a technology in which a signal is propagated by a random walk-based method. Specifically, the signal propagation of a first drug is simulated, and the signal propagation of a second drug different from the first drug is simulated in order to predict a drug-drug interaction. However, since a signal is propagated by the random walk-based method, signal propagation occurs randomly, which causes a noise problem that may lead to inaccurate results.

Accordingly, in order to solve a noise problem occurring in the signal propagation with an existing random walk-based process, the present inventors have invented a method whereby directionality is provided to signal propagation so that data can be processed with high throughput and a target protein can be predicted with high accuracy.

[Patent document]

Korean Patent Laid-open Publication No. 10-2016-0149623 (December 28, 2016)

The document ("Network Enhancement: a general method to denoise weighted biological networks", ARXIV.ORG, CORNELL UNIVERSITY LIBRARY) outlines a Network Enhancement (NE), a diffusion-based algorithm for network denoising that does not require supervision or prior knowledge. In the document it is disclosed that the NE takes as input a noisy, undirected, weighted network and outputs a network on the same set of nodes but with a new set of edge weights, the main crux of NE is that nodes connected through paths with high-weight edges are more likely to have a direct, high-weight edge between them.

The document ("Curvature graph network", International Conference on Learning Representations) outlines a network architecture that incorporates a discrete graph curvature, which measures how the neighborhoods of a pair of nodes are structurally related, wherein the curvature of an edge (x, y) is defined by comparing the distance taken to travel from neighbors of x to neighbors of y, with the length of edge (x, g).

The document ("Edge-based analysis of networks: curvatures of graphs and hypergraphs", Theory in Biosciences, vol. 139, no. 4, pages 337-348) outlines network curvatures which quantify local structural properties of (hyper) edges, that is, how, and how well, they are connected to others.

## Disclosure of Invention

## Technical Problem

The invention is defined by the appended claims.

The present disclosure provides a method and system whereby a noise problem occurring in the related art is solved because a signal is propagated with predetermined directionality rather than non-directionally in an existing random walk-based method.

The present disclosure also provides a method and system with high prediction accuracy of a target node related to a queried entity because the direction of signal propagation is biased toward nodes and edges with high importance.

The present disclosure also provides a method and system with high prediction accuracy of a target node related to a queried entity, whereby the direction of signal propagation is determined by using both the curvature of an edge and prior knowledge so that a signal can be propagated along edges and nodes with high importance.

Objectives of the present disclosure are not limited to the objectives mentioned above, and other unmentioned objectives will be clearly understood from the description below.

## Solution to Problem

According to an aspect of the present disclosure, there is provided a method for searching for a target node related to an input queried entity in a network including a plurality of nodes and edges, the method including (a) computing curvatures of edges included in the network by using a curvature computation device, and assigning a weight to each of edges based on the computed curvatures by using a weight assigning device, (b) inputting the queried entity through an input device, (c) assigning a first value to one or more main nodes corresponding to a first entity known to be related to the queried entity and assigning a second value to a node corresponding to a second entity other than the first entity by using a data assigning device, (d) propagating a signal from the main node, and propagating the signal centering on an edge having a high weight, (e) assigning a corrected weight to each of the edges included in the network by increasing the weight of the edge included in a signal propagation path in (d) and decreasing the weight of the edge that is not included in the signal propagation path by using a weight correction device, (f) assigning a third value in which association between the input queried entity and entities corresponding to the plurality of nodes constituting the network is quantified, to the plurality of nodes by using the data assigning device, and (g) propagating a signal based on the corrected weight and the third value.

The method may be a method for searching for a target node related to an input queried entity in a homogeneous network which includes a plurality of nodes belonging to a same category among categories including diseases, proteins or genes, and drugs, and a plurality of edges connecting the nodes; and wherein (b) may include inputting a queried entity belonging to a category different from that of a node constituting the homogeneous network.

The method may further include, after (g), (h) determining one or more of the nodes included in the signal propagation path in (g) as the target node; and (i) outputting an entity corresponding to the determined target node as an entity related to the queried entity through an output device.

Wherein, in (g), a node with a higher third value has a higher signal intensity starting from the corresponding node, (g) may further include propagating a signal centering on an edge having a higher corrected weight, and wherein, in (g), as the signal propagates, a data value of the node constituting the network changes, and a state in which the data value of the node does not change is defined as a signal propagation saturation state; and wherein (h) may further include determining a target node based on the data value of the node in the signal propagation saturation state.

(i) may further include outputting ranks of target nodes based on a data value of the determined target node through the output device.

When a category of the queried entity is a disease, the third value may include a value of comparative data obtained by comparing first biological data possessed by a person having a disease corresponding to the queried entity with second biological data possessed by a person without a disease corresponding to the queried entity.

The third value is a p-value indicating the significance of a degree of increase or decrease in expression for each protein or gene of a patient having a disease corresponding to the queried entity, or a degree of gene mutation of a patient having a disease corresponding to the queried entity is quantified.

When a category of the queried entity is a drug, the third value may include a change value of biological data appearing in a specific entity when the drug corresponding to the queried entity is treated to the specific entity.

The third value may be a p-value indicating the significance of a degree of expression change for each protein or gene when the drug corresponding to the queried entity is treated.

In (a), the curvature of each edge may be determined based on the numbers of neighboring nodes adjacent to nodes connected to each other through the edge.

In (a), the curvature of each edge may be determined based on the numbers of neighboring nodes adjacent to the first and second nodes connected to each other through the edge, when the number of third nodes connected to the first node through the edge is N_s, the number of fourth nodes connected to the second node through the edge is N_t and the number of intersections of the third nodes and the fourth nodes is N_i, the curvature of each edge may be determined based on N_s, N_t, and N_i.

In (a), the curvature of each edge may be determined by 4 - (N_s) - (N_t) + 3 × (N_i).

In (a), a weight assigned to each edge may be determined by a value outputted from a monotonically increasing function with curvature determined as 4 - (N_s) - (N_t) + 3 × (N_i) as a variable.

The monotonically increasing function may be

$$y = e^{\frac{\beta(k-\bar{k})}{sd(k)}}$$

where y is a weight, β is a coefficient for controlling the effect of curvature on weights, k is the curvature of the corresponding edge, $\bar{k}$ is the average of the curvatures, and sd(k) corresponds to the standard deviation of the curvatures.

The network may be a homogeneous network with a protein as a node and a relation between the protein as an edge.

The queried entity may be a disease or a drug, and the first entity may be a protein known to be related to the disease or the drug.

The queried entity may be a protein, and the first entity may be a disease or a drug known to be related to the protein.

The method may further include, before (a), (a0) extracting a homogeneous network including a node and an edge which is a relation between the nodes by extracting a node of only one type of a disease, a protein or a gene, and a drug from a heterogeneous network in which one or more of diseases, proteins, genes, and drugs are defined as nodes and a relation between the nodes is defined as an edge, by using the network extraction device.

According to another aspect of the present disclosure, there is provided a system constructed using the method described above.

According to another aspect of the present disclosure, there is provided a computer program stored in a computer-readable recording medium to execute the method described above.

## Advantageous Effects of Invention

According to the present disclosure, since a signal is propagated with predetermined directionality rather than non-directionally in a random walk-based method according to the related art, a noise problem occurring in the related art can be solved.

In addition, since the direction of signal propagation is biased toward nodes and edges with high importance, the prediction accuracy of target nodes related to a queried entity is high.

In addition, since the direction of signal propagation is determined by using both the curvature of an edge and prior knowledge and data related to a queried entity, a signal can be propagated along edges and nodes of high importance so that the prediction accuracy of a target node related to a queried entity is high.

In addition, since an algorithm according to the present disclosure showed remarkably high performance in various networks having different characteristics/ structures, the algorithm has high versatility compared to other algorithms that show performance only in a specific network.

## Brief Description of Drawings

FIG. 1 is a block diagram schematically illustrating a system according to an embodiment of the present disclosure.
FIG. 2 is a flowchart illustrating a method according to an embodiment of the present disclosure.
FIG. 3 is a view schematically illustrating the curvature of each edge in a network including nodes and edges.
FIG. 4 is a view illustrating a process according to an embodiment of the present disclosure in which a target node related to a queried entity is searched for by assigning a corrected weight to an edge constituting a network and providing directionality of signal propagation based on the assigned weight.
FIG. 5 is a view illustrating that STRING and Bio-GRID networks have different characteristics from each other.
FIGS. 6 and 7 are views showing results according to verification experiment 1.
FIG. 8 is a view showing results according to verification experiment 2.
FIG. 9 is a view showing results according to verification experiment 3.
FIG. 10 is a view showing results according to verification experiment 4.

## Mode for the Invention

Hereinafter, the present disclosure will be described in detail with reference to the accompanying drawings.

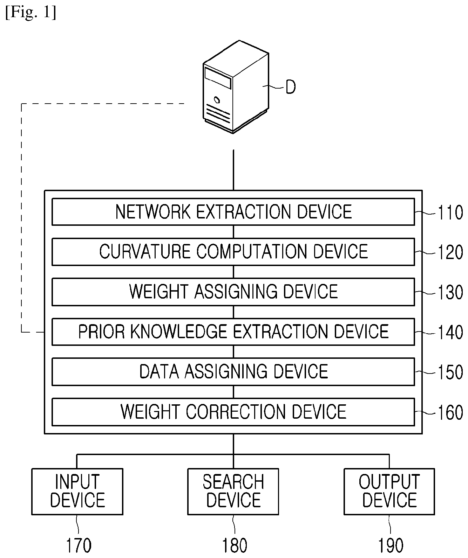

Referring to FIG. 1, a system 100 according to the present disclosure includes a network extraction device 110, a curvature computation device 120, a weight assigning device 130, a prior knowledge extraction device 140, a data assigning device 150, a weight correction device 160, an input device 170, a search device 180, and an output device 190.

The system 100 according to the present disclosure is provided with a communication device (not shown), so that mutual wired/wireless communication is possible with external databases D through a communication network.

The database D may be an external database independent of the system 100 of the present disclosure (*e.g.*, a dissertation database, a medical information database, a pharmaceutical information database, and a search portal database), and an internal database included in the system 100 of the present disclosure.

For example, the database D may be a database in which protein-protein interaction (PPI) network information is stored, and examples of the PPI network may include STRING (Nucleic Acids Res. 2005) and BioGRID (Nucleic Acids Res. 2006). However, embodiments of the present disclosure are not limited to the illustrated PPI network.

In addition, for example, heterogeneous network information including nodes and edges, in which each disease, protein or gene, or drug is defined as a node, and the relation between nodes is defined as an edge, may be stored in the database D. In the heterogeneous network, a network presented in Korean Patent Registration No. 10-2225278 by the present applicant may be included as a reference.

The network extraction device 110 is configured to extract nodes corresponding to any one type among nodes constituting the heterogeneous network stored in advance in the database D and to extract homogeneous network information including the extracted nodes and edges that are the relation between the extracted nodes.

As an example, the network extraction device 110 may extract network information such as a network including a disease node and an edge between the disease nodes, a network including a protein node and an edge between the protein nodes, or a network including a drug node and an edge between the drug nodes.

In addition, in the present disclosure, the network extraction device 110 may extract human protein-protein interaction network information, and a disease-specific network in which protein binding information in a specific disease (*e.g.*, diabetes, Alzheimer's disease, *etc.*) is added to the extracted human protein-protein interaction network information may be used.

In other words, specific information that appears in the disease/drug/protein/gene of interest (protein binding information that appears in a specific disease, protein expression information that appears when a specific drug is treated) is combined with the homogeneous network information extracted by the network extraction device 110 so as to configure a network, so that the search accuracy of a target node related to a queried entity input through the input device 170 may be increased.

The curvature computation device 120 is configured to compute the curvature of edges constituting the homogeneous network according to a predetermined method in a homogeneous network including only one type of node and an edge between the nodes.

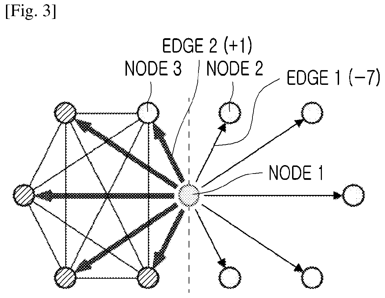

A curvature computation process using the curvature computation device 120 will be described in detail with reference to FIG. 3.

The curvature of each edge is determined according to the number of neighboring nodes adjacent to nodes connected to each other through the edge. Taking EDGE 1 as an example with reference to FIG. 3, NODE 1 and NODE 2 connected to each other through EDGE 1 may exist. The number of neighboring nodes adjacent to NODE 1 through an arbitrary edge is 10, and the number of neighboring nodes adjacent to NODE 2 through an arbitrary edge is 1. Here, the neighboring node for each node means a node connected to each node through only one edge. However, the present disclosure is not limited thereto, and nodes connected through two or more edges may also be referred to as "neighboring nodes."

The curvature computation device 120 computes the curvature of the edge through the following equation:

$$4 - (N\_s) - (N\_t) + 3 \times (N\_i),$$

where N_s represents the number of first neighboring nodes connected to NODE 1 through an edge, N_t represents the number of second neighboring nodes connected to NODE 2 through an edge, and N_i represents the number of intersections of the first neighboring nodes and the second neighboring nodes.

Taking EDGE 1 as an example, N_s = 10, N_t = 1, N_i = 0 (with respect to EDGE 1, there is no intersection between the neighboring nodes of NODE 1 and the neighboring nodes of NODE 2.).

Thus, EDGE 1 has curvature of $4 - 10 - 1 + 3 \times 0 = -7$.

EDGE 2 will be used as another example to be described. NODE 1 and NODE 3 are connected to each other through EDGE 2, the number of neighboring nodes adjacent to NODE 1 through an edge is 10, and the number of neighboring nodes adjacent to NODE 3 through an edge is 5.

Since N_s = 10, N_t = 5, and N_i = 4 (there are four (4) nodes that correspond both the neighboring nodes of NODE 1 and the neighboring nodes of NODE 3), EDGE 2 has curvature of +1 (= $4 - 10 - 5 + 3 \times 4$).

In this way, the curvature computation device 120 computes the curvature of each edge constituting the network, and the computed curvature is converted into a weight by using a predetermined method.

The weight assigning device 130 may assign a y value outputted from a monotonically increasing function (y = f(x)) using the curvature computed by the curvature computation device 120 as a variable x, as the weight of an edge. The monotonically increasing function means a function in which the y value increases as an x value increases.

As an example, the following equation may be used as the monotonically increasing function applied to the present disclosure:

$$y = e^{\frac{\beta(k - \bar{k})}{sd(k)}} \quad \cdots (1),$$

where y is a weight, $\beta$ is a coefficient for controlling the effect of curvature, k is the curvature of the corresponding edge, $\bar{k}$ is the average of the curvatures, and sd(k) corresponds to the standard deviation of the curvatures.

In a signal propagation process for searching a target node related to a queried entity, the propagation starts from a node that corresponds to an entity that is known to be related to a queried entity input through the input device 170 and falls into the same category as a node constituting a network (hereinafter, referred to as a "main node"). The propagation is performed from one node to another with a probability that depends on the weight of an edge connecting the nodes. For example, the higher the edge weight assigned by the weight assigning device 130 is, the higher the probability that a signal will be propagated along the edge is. That is, in the present disclosure, by giving directionality to a signal propagation based on the weight assigned by the weight assigning device 130, the target node can be searched for with higher accuracy compared to the related art in which the same weights are assigned to all edges to perform signal propagation.

The prior knowledge extraction device 140 is configured to extract information (entities) known to be related to the queried entity input through the input device 170. For example, the prior knowledge extraction device 140 is configured to extract "a queried entity - an entity known to be related to the queried entity among entities corresponding to the same type as the type of nodes constituting the homogeneous network (for example, in the case of a homogeneous network including only of protein nodes, the category of the extracted entity is a protein)" in a plurality of papers stored in advance in the database D.

For example, when disease A is input as a queried entity and the database D includes information "the probability of inducing disease A may increase due to denaturation of protein B," the prior knowledge extraction device 140 may extract protein B as prior knowledge related to disease A.

The data assigning device 150 is configured to assign a first value to a node corresponding to the prior knowledge extracted by the prior knowledge extraction device 140 and to assign a second value to a node that does not correspond to the prior knowledge. Here, the first value may be "1" and the second value may be "0," but as long as the first value and the second value are distinguishable from each other, embodiments of the present disclosure are not limited thereto.

That is, the data assigning device 150 assigns a first value (e.g., 1) to the main node corresponding to a first entity known to be related to the queried entity and assigns a second value (e.g., 0) to a node corresponding to a second entity other than the first entity.

In other words, the first value is assigned to an entity that is known to be related to the queried entity, and the second value is assigned to an entity that is not known to be related to the queried entity, so that a signal is propagated from the main node in a signal propagation process. That is, the probability that a signal propagation along nodes corresponding to the entity known to be related to the queried entity increases, and the target node can be searched for with higher accuracy than in the related art.

The weight correcting apparatus 160 serves to correct weights assigned to edges by increasing the weights of edges included in the signal propagation path and decreasing the weights of edges that are not included in the signal propagation path.

When the weights are assigned to the edges constituting the network by the weight assigning device 130 and the first and second values are assigned to the nodes constituting the network by the data assigning device 150, first signal propagation starting from the main node may be performed. The first signal propagation may be performed with the main node as a starting point in such a way that the higher the weight assigned to the edge by the weight assigning device 130 is, the higher the probability of signal propagation along the corresponding edge is.

When the first signal propagation is completed (reaches a saturated state ), the weight correction device 160 increases the weight of the edge included in a first signal propagation path and decreases the weight of the edge not included in the first signal propagation path. Thus, the corrected weight is assigned to each edge constituting the homogeneous network by the weight correction device 160.

As the first signal propagation starting from the main node proceeds (diffusion), the data values of nodes change (e.g., even though a neighboring node adjacent to the main node had the second value at the beginning of the signal propagation, the data value of the neighboring node may change to a value between the first value and the second value as the signal propagation proceeds). When signal propagation is performed in the network, the data value may change in a direction in which the data value of a node increases in proportion to the number of times at which a signal passes through the node in the signal propagation process. The weight of an edge is readjusted using the data value of a node when the data value of the node no longer changes, that is, when the signal propagation is completed (signal propagation saturation) (e.g., in a way whereby a specific data value is assigned to the edge included in the first signal propagation path). The signal propagation saturation means a state in which the data value no longer changes even when time has elapsed.

In addition, the data assigning device 150 is configured to assign a third value in which association between the input queried entity and the entities corresponding to a plurality of nodes constituting the network is quantified, to the nodes forming the network.

For example, when the queried entity is a disease, for example, the third value may be a value of comparative data obtained by comparing first biological data possessed by a person having a disease corresponding to the queried entity with one or more of second biological data possessed by a person without the disease corresponding to the queried entity or a person having a disease other than the disease corresponding to the queried entity.

More specifically, the third value may be derived by comparing the first biological data possessed by a person having the disease corresponding to the queried entity with the second biological data possessed by a normal person without the disease, by comparing the first biological data with the second biological data possessed by a person with a disease other than the disease, or by comparing the first biological data with summarized data obtained by adding the biological data possessed by a normal person without the disease to the biological data possessed by a person with a disease other than the disease. However, embodiments of the present disclosure are not limited to the above-described example, and all values that can be derived by comparing the first biological data with one or more of the second biological data may be included in the third value.

Here, biological data may include all biological information possessed by a specific entity, such as gene information, gene expression information, protein information, and protein expression information. That is, biological data may include all biological information that may be quantified, and examples of the biological data may include various levels of omics data such as genomics information, transcriptomics information, proteomics information, and metabolomics information, and optical values using fluorescent proteins. More specifically, the third value may be a value in which association between a queried entity and a plurality of nodes constituting the network is quantified, such as a p-value indicating the significance of the degree of increase or decrease in expression for each protein/gene in the patient's protein or gene expression data compared to a normal person and a value indicating the degree of gene mutation. The quantified third value may be assigned to each of the plurality of nodes.

In another example, when the queried entity is a drug, for example, the third value may be the value of change in biological data that appears in a specific entity when the drug corresponding to the queried entity is treated to the specific entity. A p-value indicating the significance of the degree of change in gene or protein expression when the drug is treated may correspond to the third value.

The input device 170 may be a touch panel, a keyboard, or a scanner, but embodiments of the present disclosure are not particularly limited thereto as long as the input device 170 can receive a user command and transmit the command to the system according to the present disclosure.

The search device 180 is configured to search an entity corresponding to a node included in a second signal propagation path as an entity related to the queried entity by allowing a signal to propagate (second signal propagation) centering on edges having high corrected weights and nodes having high third values.

Here, propagation of a signal centering on a node having a high third value means that the magnitude of the third value assigned to the node affects the intensity of the signal. Although all nodes constituting the network can be a starting point of signal propagation (meaning that signal propagation starting from each node can be performed with its own intensity), a node with a higher third value has a higher signal intensity starting from the node, and thus signal propagation can be made centering on the node to which the high third value is assigned.

Specifically, as the second signal propagation proceeds (diffusion), a data value of a node changes until the signal propagation reaches a saturated state. Target nodes may be determined using data values of nodes in a state in which signal propagation is saturated, and ranks of the target nodes may be determined based on the data values. That is, a target node with a higher data value may correspond to a high-ranking target node, and a target node with a lower data value may correspond to a low-ranking target node. Entities corresponding to the target nodes determined by the search device 180 may be listed based on the determined ranks and outputted through the output device 190. Thus, it is possible for a system user to intuitively check the entities corresponding to the target nodes related to the queried entity in order of importance.

The output device 190 may be a monitor or a display panel, but embodiments of the present disclosure are not particularly limited thereto as long as a computation result of the system according to the present disclosure can be visually checked by the output device 190.

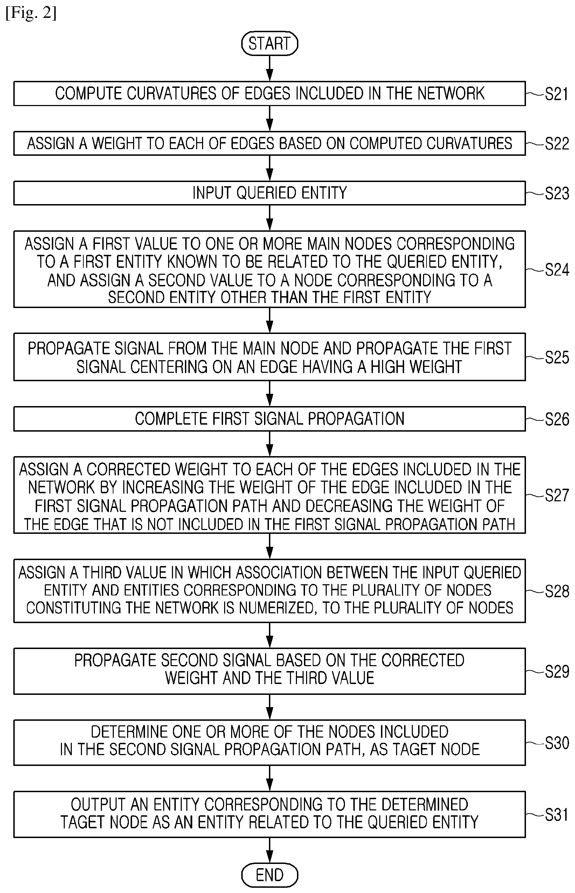

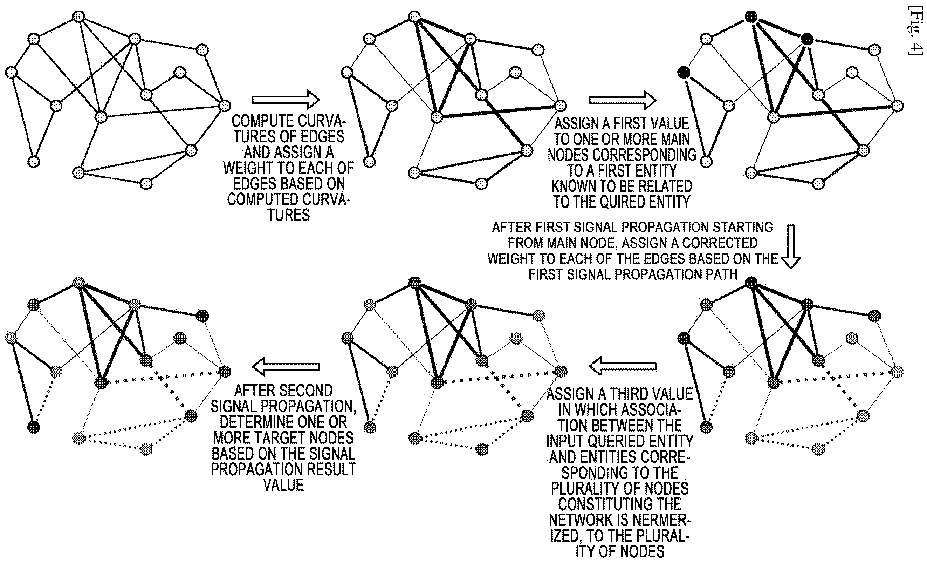

Next, a method according to an embodiment of the present disclosure will be described in detail with reference to FIGS. 2 and 4.

First, the curvature of each of the edges included in a network is computed by the curvature computation device 120 (S21). The network may be a homogeneous network including only one type of entity (e.g., a disease node, a protein or gene node, or a drug node) and the relation between these entities (edge). In another embodiment, a process of extracting a homogeneous network by the network extraction device 110 from a heterogeneous network stored in advance in a database D or the like may be preceded. In another embodiment, the network may be formed by combining specific information that appears in the disease/drug/protein/gene of interest (e.g., protein binding information in a specific disease, protein expression information when a specific drug is treated), with a homogeneous network.

The curvature of each edge may be determined based on the number of neighboring nodes adjacent to nodes connected through the edge, and a description thereof has been described above.

Next, according to the curvature computed by the curvature computation device 120, the weight assigning device 130 assigns a weight to each of the edges included in the network (S22). When weights are assigned to the edges, the network may have the form of the upper center drawing of FIG. 4, and the weights assigned to the edges are y values outputted from a monotonically increasing function ($y = f(x)$) with curvature as the variable x. It means that the higher the curvature, the higher the weight assigned.

Next, a queried entity is input through the input device 170 (S23). The queried entity may be a disease or a drug when the network includes a protein node and an edge corresponding to a relation between the protein nodes, but embodiments of the present disclosure are not limited thereto as long as the queried entity may be any entity related to the protein. The queried entity may have any one category of disease, protein, drug, and gene.

Next, the prior knowledge extraction device 140 extracts an entity that is known to be related to the input queried entity and falls under the same category as the node constituting the network (for example, a protein when the node constituting the network is a protein), from prior knowledge data stored in the database D. There may be a plurality of entities known in advance to be related to the queried entity, and all of the plurality of entities may be extracted.

Next, the data assigning device 150 assigns a first value to a node (main node) corresponding to a first entity extracted by the prior knowledge extraction device 140, and assigns a second value to a node corresponding to an entity other than the first entity (a second entity) (S24) *(see* the upper right drawing of FIG. 4).

Next, a signal is propagated from the main node with a probability that depends on the edge weight, for example, with a probability that is proportional to the weight of the edge connecting nodes. A first signal is propagated with directionality centering on an edge with a high weight (S25). That is, the higher the weight of an edge is, the more the signal propagates along the edge.

When the first signal propagation is completed (reaches a saturated state) (S26), the weight correction device 160 increases the weights of edges included in the first signal propagation path, and decreases the weights of edges that are not included in the first signal propagation path, thereby correcting the weights of the edges included in the network (S27). That is, corrected weights are assigned to the edges included in the network by the weight correction device 160 *(see* the lower right drawing of FIG. 4).

Next, the data assigning device 150 assigns a third value in which the association between the input queried entity and the entities corresponding to a plurality of nodes constituting the network is quantified, to the nodes constituting the network (S28). For example, when the queried entity is a disease, the third value may be computed as a form of a p-value indicating the significance of the degree of increase or decrease in expression for each protein/gene in the patient's protein or gene expression data compared to a normal person or a value in which the degree of gene mutation of the patient is quantified, and the computed third value may be assigned to each of the plurality of nodes. In another example, when the queried entity is a drug, a p-value indicating the significance of the degree of change in gene or protein expression when the drug is treated may correspond to the third value *(see* the lower center drawing of FIG. 4).

Next, a second signal is propagated centering on an edge having a high corrected weight and a node having a high third value (S29). Nodes included in the second signal propagation path may be determined as target nodes (S30), and entities corresponding to the determined target nodes may be outputted through the output device 190 as entities related to the queried entity (S31). In another example, the ranks of the target nodes may be determined using a signal propagation result value (*e.g.,* a data value of a node in a state in which signal propagation is saturated), and the target nodes may be listed and outputted to the output device 190 in descending order from a target node having the highest rank to a target node having the lowest rank.

Verification Experiment 1

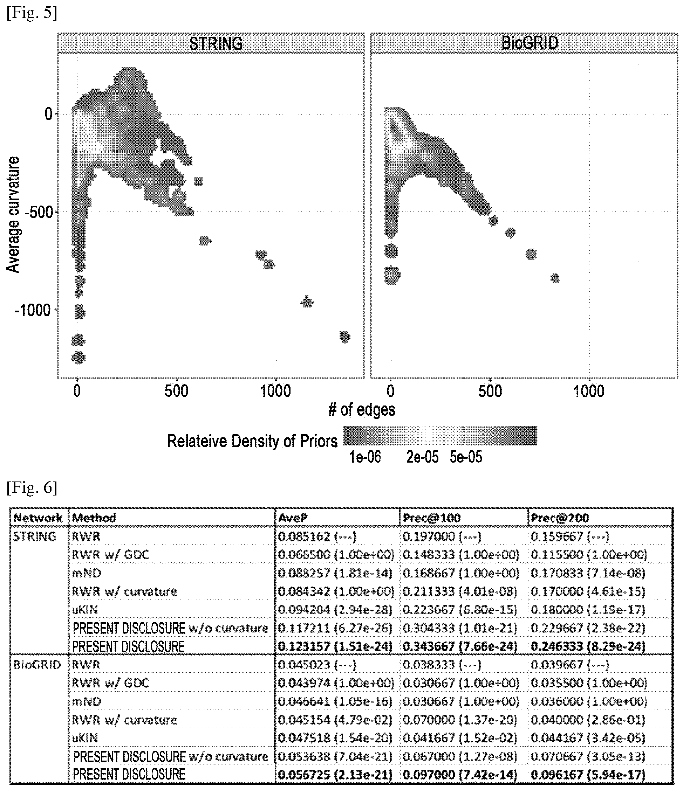

A verification experiment was conducted to verify the enhanced performance of the method according to the present disclosure. PPI networks of STRING (Nucleic Acids Res. 2005) and BioGRID (Nucleic Acids Res. 2006) were used in the verification experiment. By examining the average of the edge curvature and the number of edges connecting the nodes constituting the network, it was confirmed that the characteristics of the STRING and BioGRID networks were significantly different *(see* FIG. 5).

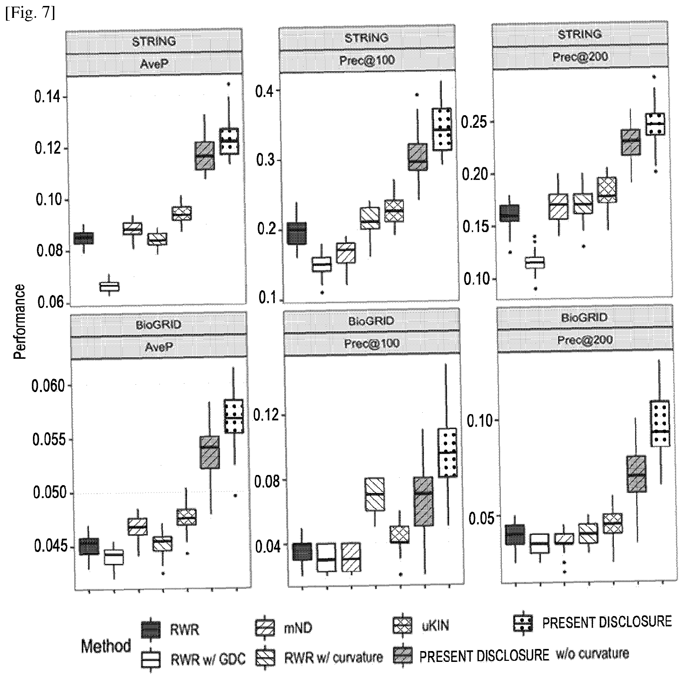

In each of STRING network and BioGRID network, average precision (AveP), Prec@ 100 and Prec@200 (prediction accuracy up to the top 100, 200 ranks) was computed for each of a Random Walk with Restart (RWR) method, an RWR method with GDC (NeurIPS 2019) applied (RWR w/ GDC), an mND (Bioinformatics 2020) method, a uKIN (RECOMB 2020) method, a method in which only weights using the curvature of an edge are assigned and no node data according to prior knowledge is assigned (RWR w/ curvature), a method to which only node data according to prior knowledge is assigned and weights using the curvature of an edge are not assigned (present disclosure w/o curvature), and the method according to the present disclosure, respectively. As a result of the experiment, it was confirmed that the method of the present disclosure had the highest performance for both STRING and BioGRID networks *(see* FIGS. 6 and 7).

That is, it was confirmed through the verification experiment that the method according to the present disclosure is applicable to various networks having different characteristics to each other, and has a remarkably high performance even when it is applied to any network.

Verification Experiment 2

A verification experiment was conducted to evaluate the effect of the curvature itself applied to an edge when a weight using the curvature of the edge was assigned.

For each of the RWR method, the RWR w/ GDC (NeurIPS 2019) method, and the RWR w/ curvature method (RWR method to which a weight using the curvature of an edge is applied), an experiment was conducted to search for a target node related to a queried entity while adjusting $\beta$ (beta) *(see* Equation 1), which is a coefficient affecting the weight assigned to an edge according to the curvature.

In the case of the RWR w/ GDC method, it was confirmed that the performance was constant regardless of the value of $\beta$ because it does not include an algorithm that assigns weights according to the curvature of an edge.

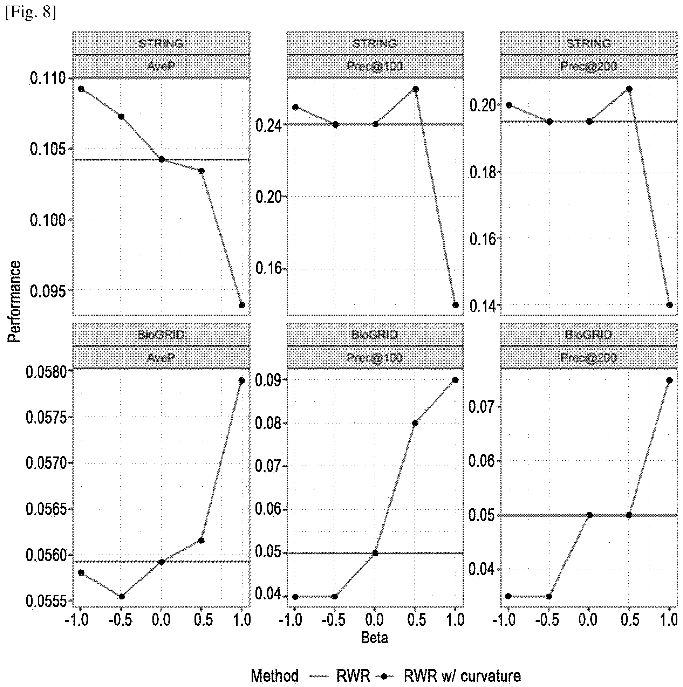

In the case of the RWR w/ curvature method (a method in which only weights using the curvature of an edge are assigned and no node data according to prior knowledge is assigned), in an experiment using a STRING network, it was confirmed that the performance deteriorated as $\beta$ was a large positive value, and rather, the performance slightly increased when $\beta$ was a negative value (when the curvature effect was reversed), compared to the RWR method (β = 0). On the other hand, in an experiment using a BioGRID network, the performance slightly decreased when β had a negative value (when the curvature effect was reversed), and the performance increased as β had a large positive value, compared to the RWR method (β = 0) *(see* FIG. 8).

In other words, unlike the present disclosure, when applying an algorithm that only assigns weights using the curvature of an edge, it was confirmed that the type of the network to which the algorithm is applied was limited in that the performance varies depending on the structure of the network used.

Verification Experiment 3

Using the method according to the present disclosure, a verification experiment was conducted to evaluate the effect of β, while varying the coefficient β that affects the weight assigned to an edge based on the curvature (β = -1, -0.5, 0, +0.5 or +1).

β is a coefficient applied to the monotonically increasing function *(see* Equation 1). As the value of β changes, the rate of change of the weight assigned to an edge changes (that is, β corresponds to a parameter for controlling the effect of the edge curvature).

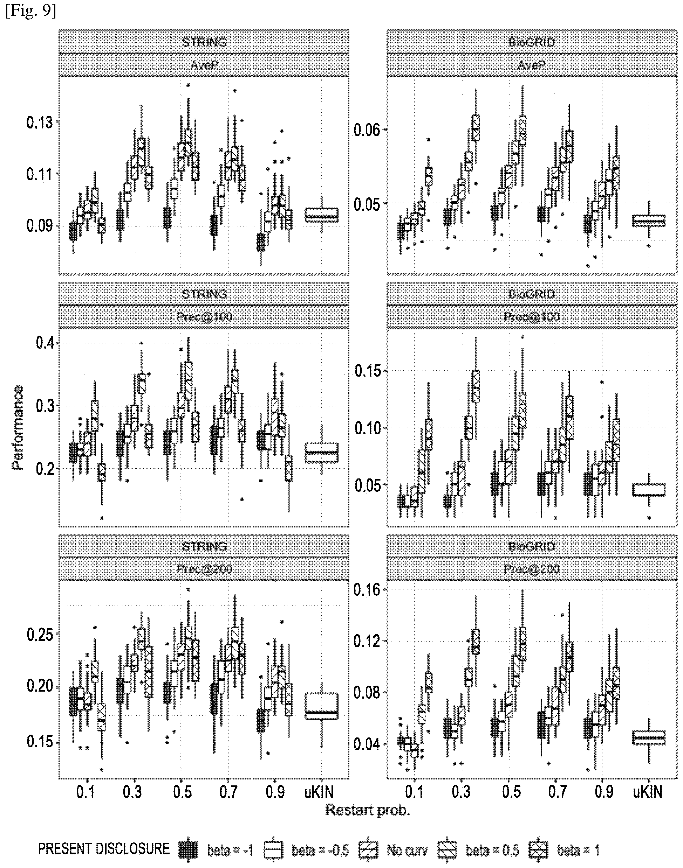

As a result of the experiment, the highest prediction performance was shown with any evaluation method when β was 0.5 in the experiment using the STRING network, and when β was 1 in the experiment using the BioGRID network *(see* FIG. 9).

Verification Experiment 4

An experiment was conducted to verify the enhanced performance of the method according to the present disclosure, even when all prior knowledge of a database was not utilized.

By using only 0.05%, 0.1%, 0.15%, and 0.2% of the data stored in the database D, the prediction performance of the method according to the present disclosure and that of the uKIN (RECOMB 2020) method (a method utilizing only prior knowledge without assigning weights of edges) were evaluated. The present disclosure, including both a weight assigning process based on the curvature of the edge and a node data assigning process based on the prior knowledge, showed higher prediction performance than the related art including only one process.

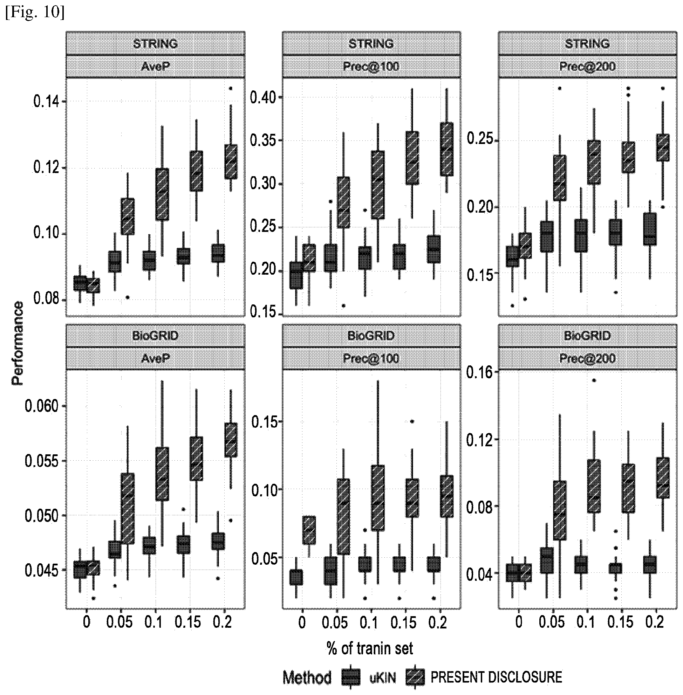

As a result of the experiment, for both STRING and BioGRID networks, the method according to the present disclosure showed higher prediction performance compared to the related art (uKIN), even if much prior knowledge was not used. Also, it was confirmed that the prediction performance increased linearly as the amount of prior knowledge increased *(see* FIG. 10).

All or at least a part of the configuration of the system according to the embodiment of the present disclosure may be implemented in the form of a hardware module or a software module, or may be implemented in a form in which a hardware module and a software module are combined.

Here, the software module may be understood as, for example, instructions executed by a processor that controls operations in the system, and these instructions may have a form mounted in a memory in the target node prediction system.

The method for searching for a target node according to the present disclosure described above may be implemented in the form of program instructions that can be executed through various computer means and recorded in a computer-readable medium. The computer-readable medium may include program instructions, data files, data structures, *etc.,* alone or in combination. The program instructions recorded on the medium may be specially designed and configured for the present disclosure, or may be known and available to those skilled in the art of computer software. Examples of the computer-readable recording medium include magnetic media such as hard disks, floppy disks, and magnetic tapes, optical media such as CD-ROMs and DVDs, magneto-optical media such as floptical disks, or hardware devices specifically configured to store and execute program instructions, such as ROM, RAM, and flash memory, and the like. Examples of program instructions include not only machine language codes such as those generated by a compiler, but also high-level language codes that can be executed by a computer using an interpreter or the like. The hardware devices described above may be configured to operate as one or more software modules to carry out the operations of the present disclosure, and vice versa.

While the present invention has been particularly shown and described with reference to exemplary embodiments thereof, it will be understood by those of ordinary skill in the art that various changes in form and details may be made therein without departing from the scope of the present invention as defined by the following claims.

(Explanation of reference numerals)

    100: system
    110: network extraction device
    120: curvature computation device
    130: weight assigning device
    140: prior knowledge extraction device
    150: data assigning device
    160: weight correction device
    170: input device
    180: search device
    190: output device
    D: database

**Claims**

1. A computer-implemented method for searching for target nodes related to an input queried entity in a network including a plurality of nodes and edges, each of the plurality of nodes denoting a disease, a protein, a gene, or a drug, the method comprising:

   (a) computing curvatures of edges included in the network by using a curvature computation device (120), and assigning a weight to each of edges based on the computed curvatures by using a weight assigning device (130);
   (b) inputting the queried entity through an input device (170);
   (c) assigning a first value, such as 1, to a main node corresponding to a first entity known to be related to the queried entity, and assigning a second value, such as 0, to a node corresponding to a second entity other than the first entity by using a data assigning device (150);
   (d) propagating a first signal from the main node, wherein the first signal is propagated while having a directionality along an edge having a higher weight;
   (e) assigning a corrected weight to each of the edges included in the network by increasing the weight of the edge included in a signal propagation path in (d) and decreasing the weight of the edge that is not included in the signal propagation path by using a weight correction device (160);
   (f) assigning a third value which has been quantified from association between the input queried entity and entities corresponding to the plurality of nodes constituting the network, such as, a p-value indicating significance of degree of increase or decrease in expression for each protein/gene in a patient's protein or gene expression data compared to a normal person, or a value indicating degree of gene mutation, to the plurality of nodes by using the data assigning device (150);
   (g) propagating a second signal based on the corrected weight and the third value;
   (h) determining nodes included in the propagation path of the second signal in (g) as the target nodes; and
   (i) outputting entities corresponding to the determined target nodes as entities related to the queried entity through an output device (190).

2. The computer-implemented method of claim 1, wherein the method is a method for searching for a target node related to an input queried entity in a homogeneous network which includes a plurality of nodes belonging to a same category among categories including diseases, proteins or genes, and drugs, and a plurality of edges connecting the nodes; and wherein (b) comprises inputting a queried entity belonging to a category different from that of a node constituting the homogeneous network.

3. The computer-implemented method of claim 1, wherein, in (g), a node with a higher third value has a higher signal intensity starting from the corresponding node, wherein the second signal is propagated while having a directionality along on an edge having a higher corrected weight; wherein, in (g), as the second signal propagates, a data value of the node of the network changes, and a state in which the data value of the node does not change is defined as a signal propagation saturation state; wherein (h) further comprises determining the target nodes based on data values of the nodes in the signal propagation saturation state, wherein the data value changes in a direction in which the data value of the node increases in proportion to the number of times at which the second signal passes through the node in the signal propagation process.

4. The computer-implemented method of claim 1, wherein, when a category of the queried entity is a disease, the third value includes a value of comparative data obtained by comparing first biological data possessed by a person having a disease corresponding to the queried entity with second biological data possessed by a person without a disease corresponding to the queried entity.

5. The computer-implemented method of claim 4, wherein the third value is a p-value indicating the significance of a degree of increase or decrease in expression for each protein or gene of a patient having a disease corresponding to the queried entity, or a value in which a degree of gene mutation of a patient having a disease corresponding to the queried entity is quantified.

6. The computer-implemented method of claim 1, wherein, when a category of the queried entity is a drug, the third value includes a change value of biological data appearing in a specific entity when the specific entity is treated by the drug corresponding to the queried entity.

7. The computer-implemented method of claim 6, wherein the third value is a p-value indicating the significance of a degree of expression change for each protein or gene when the specific entity is treated by the drug corresponding to the queried entity.

8. The computer-implemented method of claim 1, wherein, in (a), the curvature of each edge is determined based on numbers of neighboring nodes adjacent to the first and second nodes connected to each other through the edge, when the number of third nodes connected to the first node through the edge is N_s, the number of fourth nodes connected to the second node through the edge is N_t and the number of intersections of the third nodes and the fourth nodes is N_i, the curvature of each edge is determined by 4 - (N_s) - (N_t) + 3 x (N_i).

9. The computer-implemented method of claim 8, wherein, in (a), a weight assigned to each edge is determined by a value outputted from a monotonically increasing function with curvature determined as 4 - (N_s) - (N_t) + 3 x (N_i) as a variable.

10. The computer-implemented method of claim 9, wherein the monotonically increasing function is

$$y = e^{\frac{\beta(k-\bar{k})}{sd(k)}}$$

where y is a weight, $\beta$ is a coefficient for controlling the effect of curvature on weights, k is the curvature of the corresponding edge, $\bar{k}$ is the average of the curvatures, and sd(k) corresponds to the standard deviation of the curvatures.

11. The computer-implemented method of claim 1, wherein the network is a homogeneous network including protein nodes and a relation between the protein nodes as an edge, the queried entity is a disease or a drug, and the first entity is a protein known to be related to the disease or the drug.

12. The method of claim 1, further comprising, before (a), (a0) extracting a homogeneous network including nodes and an edge which is a relation between the nodes by extracting a node of only one type of a disease, a protein or a gene, and a drug from a heterogeneous network in which one or more of diseases, proteins, genes, and drugs are defined as nodes and a relation between the nodes is defined as an edge, by using the network extraction device.

13. A system (100) constructed using the method of any one of claims 1 to 12.

14. A non-transitory computer readable program product to execute the method of any one of claims 1 to 12.

**Patentansprüche**

1. Computerimplementiertes Verfahren zum Suchen nach Zielknoten mit Bezug auf eine eingegebene abgefragte Einheit in einem Netzwerk, das eine Mehrzahl von Knoten und Kanten umfasst, wobei jeder der Mehrzahl von Knoten eine Krankheit, ein Protein, ein Gen oder ein Arzneimittel bezeichnet, wobei das Verfahren umfasst:

(a) Berechnen von Krümmungen von im Netzwerk enthaltenen Kanten unter Verwendung einer Krümmungsberechnungsvorrichtung (120) und Zuweisen eines Gewichts zu jeder von Kanten basierend auf den berechneten Krümmungen unter Verwendung einer Gewichtszuweisungsvorrichtung (130);
(b) Eingeben der abgefragten Einheit über eine Eingabevorrichtung (170);
(c) Zuweisen eines ersten Werts, wie etwa 1, zu einem Hauptknoten entsprechend einer ersten Einheit, von der bekannt ist, dass sie mit der abgefragten Einheit in Beziehung steht, und Zuweisen eines zweiten Werts, wie etwa 0, zu einem Knoten entsprechend einer von einer ersten Einheit verschiedenen zweiten Einheit unter Verwendung einer Datenzuweisungsvorrichtung (150);
(d) Ausbreiten eines ersten Signals vom Hauptknoten, wobei das erste Signal mit einer Direktionalität entlang einer Kante mit einem höheren Gewicht ausgebreitet wird;
(e) Zuweisen eines korrigierten Gewichts zu jeder der im Netzwerk enthaltenen Kanten durch Erhöhen des Gewichts der in (d) in einem Signalausbreitungspfad enthaltenen Kante und Verringern des Gewichts der Kante, die nicht im Signalausbreitungspfad enthalten ist, unter Verwendung einer Gewichtskorrekturvorrichtung (160);
(f) Zuweisen eines dritten Werts, der aus einer Verbindung zwischen der eingegeben abgefragten Einheit und Einheiten, die der Mehrzahl von Knoten entsprechen, die das Netzwerk bilden, quantifiziert wurde, wie etwa eines p-Werts, der eine Signifikanz eines Ausmaßes einer Zunahme oder Abnahme einer Expression für jedes Protein/Gen in Protein- oder Genexpressionsdaten eines Patienten im Vergleich zu einer normalen Person angibt, oder eines Werts, der ein Ausmaß einer Genmutation angibt, zu der Mehrzahl von Knoten unter Verwendung der Datenzuweisungsvorrichtung (150);
(g) Ausbreiten eines zweiten Signals basierend auf dem korrigierten Gewicht und dem dritten Wert;
(h) Bestimmen von Knoten, die im Ausbreitungspfad des zweiten Signals in (g) enthalten sind, als die Zielknoten und
(i) Ausgeben von Einheiten entsprechend den bestimmten Zielknoten als Einheiten mit Bezug auf die abgefragte Einheit über eine Ausgabevorrichtung (190).

**2.** Computerimplementiertes Verfahren nach Anspruch 1, wobei das Verfahren ein Verfahren zum Suchen nach einem Zielknoten mit Bezug auf eine eingegebene abgefragte Einheit in einem homogenen Netzwerk ist, das eine Mehrzahl von Knoten, die zu einer gleichen Kategorie unter Kategorien wie Krankheiten, Proteine oder Gene und Arzneimittel gehören, und eine Mehrzahl von Kanten umfasst, die die Knoten verbinden; und wobei (b) ein Eingeben einer abgefragten Einheit umfasst, die zu einer Kategorie gehört, die von jener eines Knotens verschieden ist, der das homogene Netzwerk bildet.

**3.** Computerimplementiertes Verfahren nach Anspruch 1, wobei in (g) ein Knoten mit einem höheren dritten Wert ausgehend vom entsprechenden Knoten eine höhere Signalintensität aufweist, wobei das zweite Signal mit einer Direktionalität entlang einer Kante mit einem höheren korrigierten Gewicht ausgebreitet wird; wobei sich in (g) bei Ausbreitung des zweiten Signals ein Datenwert des Knotens des Netzwerks ändert und ein Zustand, in dem sich der Datenwert des Knotens nicht ändert, als ein Signalausbreitungssättigungszustand definiert wird; wobei (h) weiterhin ein Bestimmen der Zielknoten basierend auf Datenwerten der Knoten im Signalausbreitungssättigungszustand umfasst, wobei sich der Datenwert in einer Richtung ändert, in der der Datenwert des Knotens proportional zur Anzahl der Durchläufe des zweiten Signals durch den Knoten im Signalausbreitungsprozess zunimmt.

**4.** Computerimplementiertes Verfahren nach Anspruch 1, wobei, wenn eine Kategorie der abgefragten Einheit eine Krankheit ist, der dritte Wert einen Wert von Vergleichsdaten umfasst, die durch Vergleichen erster biologischer Daten, die eine Person mit einer der abgefragten Einheit entsprechenden Krankheit besitzt, mit zweiten biologischen Daten, die eine Person ohne eine der abgefragten Einheit entsprechende Krankheit besitzt, erhalten werden.

**5.** Computerimplementiertes Verfahren nach Anspruch 4, wobei der dritte Wert ein p-Wert, der die Signifikanz eines Ausmaßes einer Zunahme oder Abnahme einer Expression für jedes Protein oder Gen eines Patienten mit einer der abgefragten Einheit entsprechenden Krankheit angibt, oder ein Wert ist, in dem ein Ausmaß einer Genmutation eines Patienten mit einer der abgefragten Einheit entsprechenden Krankheit quantifiziert wird.

**6.** Computerimplementiertes Verfahren nach Anspruch 1, wobei, wenn eine Kategorie der abgefragten Einheit ein Arzneimittel ist, der dritte Wert einen Änderungswert biologischer Daten umfasst, die in einer spezifischen Einheit auftreten, wenn die spezifische Einheit mit dem der abgefragten Einheit entsprechenden Arzneimittel behandelt wird.

**7.** Computerimplementiertes Verfahren nach Anspruch 6, wobei der dritte Wert ein p-Wert ist, der die Signifikanz eines Ausmaßes einer Expressionsänderung für jedes Protein oder Gen angibt, wenn die spezifische Einheit mit dem der abgefragten Einheit entsprechenden Arzneimittel behandelt wird.

**8.** Computerimplementiertes Verfahren nach Anspruch 1, wobei in (a) die Krümmung jeder Kante basierend auf Anzahlen benachbarter Knoten bestimmt wird, die an die ersten und zweiten Knoten angrenzen, die durch die Kante miteinander verbunden sind, wobei, wenn die Anzahl dritter Knoten, die durch die Kante mit dem ersten Knoten verbunden sind, $N\_s$ ist, die Anzahl vierter Knoten, die durch die Kante mit dem zweiten Knoten verbunden sind, $N\_t$ ist und die Anzahl von Schnittpunkten der dritten Knoten und der vierten Knoten $N\_i$ ist, die Krümmung jeder Kante durch $4 - (N\_s) - (N\_t) + 3 \times (N\_i)$ bestimmt wird.

**9.** Computerimplementiertes Verfahren nach Anspruch 8, wobei in (a) ein jeder Kante zugewiesenes Gewicht durch einen Wert bestimmt wird, der von einer monoton zunehmenden Funktion mit einer Krümmung ausgegeben wird, die als $4 - (N\_s) - (N\_t) + 3 \times (N\_i)$ als eine Variable bestimmt wird.

**10.** Computerimplementiertes Verfahren nach Anspruch 9, wobei die monoton zunehmende Funktion ist:

$$y = e^{\frac{\beta(k-\bar{k})}{sd(k)}}$$

wobei y ein Gewicht ist, $\beta$ ein Koeffizient zur Steuerung der Auswirkung einer Krümmung auf Gewichte ist, k die Krümmung der entsprechenden Kante ist, $\bar{k}$ der Durchschnitt der Krümmungen ist und $sd(k)$ der Standardabweichung der Krümmungen entspricht.

**11.** Computerimplementiertes Verfahren nach Anspruch 1, wobei das Netzwerk ein homogenes Netzwerk ist, das Proteinknoten und eine Beziehung zwischen den Proteinknoten als eine Kante umfasst, die abgefragte Einheit eine Krankheit oder ein Arzneimittel ist und die erste Einheit ein Protein ist, von dem bekannt ist, dass es mit der Krankheit oder dem Arzneimittel in Zusammenhang steht.

**12.** Verfahren nach Anspruch 1, weiterhin umfassend, vor (a), (a0) Extrahieren eines homogenen Netzwerks mit Knoten und einer Kante, die eine Beziehung zwischen den Knoten darstellt, indem unter Verwendung der Netzwerkextraktionsvorrichtung ein Knoten von nur einem Typ einer Krankheit, eines Proteins oder eines Gens und eines Arzneimittels aus einem heterogenen Netzwerk extrahiert wird, in dem eine oder mehrere Krankheiten, Proteine, Gene und Arzneimittel als Knoten definiert sind und eine Beziehung zwischen den Knoten als eine Kante definiert ist.

13. System (100), das unter Verwendung des Verfahrens nach einem der Ansprüche 1 bis 12 aufgebaut ist.

14. Nichtflüchtiges computerlesbares Programmprodukt zum Ausführen des Verfahrens nach einem der Ansprüche 1 bis 12.

**Revendications**

1. Procédé mis en œuvre par ordinateur pour rechercher des nœuds cibles relatifs à une entité d'entrée interrogée dans un réseau comprenant une pluralité de nœuds et d'arêtes, chacun de la pluralité de nœuds représentant une maladie, une protéine, un gène ou un médicament, le procédé comprenant :

(a) le calcul des courbures des arêtes incluses dans le réseau à l'aide d'un dispositif de calcul des courbures (120) et l'attribution d'un poids à chacune des arêtes sur la base des courbures calculées à l'aide d'un dispositif d'attribution de poids (130) ;
(b) l'entrée de l'entité interrogée par l'intermédiaire d'un dispositif d'entrée (170) ;
(c) l'attribution d'une première valeur, telle que 1, à un nœud principal correspondant à une première entité connue pour être liée à l'entité interrogée, et l'attribution d'une deuxième valeur, telle que 0, à un nœud correspondant à une deuxième entité autre que la première entité à l'aide d'un dispositif d'attribution de données (150) ;
(d) la propagation d'un premier signal à partir du nœud principal, le premier signal étant propagé tout en ayant une direction le long d'une arête ayant un poids plus élevé ;
e) l'attribution d'un poids corrigé à chacune des arêtes incluses dans le réseau par augmentation du poids de l'arête incluse dans un chemin de propagation de signal dans (d) et par diminution du poids de l'arête qui n'est pas incluse dans le chemin de propagation de signal à l'aide d'un dispositif de correction de poids (160) ;
(f) l'attribution d'une troisième valeur qui a été quantifiée à partir de l'association entre l'entité d'entrée interrogée et les entités correspondant à la pluralité de nœuds constituant le réseau, telle qu'une valeur p indiquant l'importance du degré d'augmentation ou de diminution de l'expression de chaque protéine/gène dans les données d'expression de protéines ou de gènes d'un patient par rapport à une personne normale, ou une valeur indiquant le degré de mutation d'un gène, à la pluralité de nœuds à l'aide du dispositif d'attribution de données (150) ;
(g) la propagation d'un deuxième signal basé sur le poids corrigé et la troisième valeur ;
(h) la détermination des nœuds inclus dans le chemin de propagation du second signal dans (g) comme étant les nœuds cibles ; et
(i) la fourniture d'entités correspondant aux nœuds cibles déterminés en tant qu'entités liées à l'entité interrogée par l'intermédiaire d'un dispositif de sortie (190).

2. Procédé mis en œuvre par ordinateur selon la revendication 1, dans lequel le procédé est un procédé de recherche d'un nœud cible lié à une entité d'entrée interrogée dans un réseau homogène qui comprend une pluralité de nœuds appartenant à une même catégorie parmi les catégories comprenant les maladies, les protéines ou les gènes, et les médicaments, et une pluralité d'arêtes reliant les nœuds ; et dans lequel (b) comprend l'entrée d'une entité interrogée appartenant à une catégorie différente de celle d'un nœud constituant le réseau homogène.

3. Procédé mis en œuvre par ordinateur selon la revendication 1, dans lequel, dans (g), un nœud ayant une troisième valeur plus élevée a une intensité de signal plus élevée à partir du nœud correspondant, dans lequel le second signal est propagé tout en ayant une directionnalité le long d'une arête bord ayant un poids corrigé plus élevé ; dans lequel, dans (g), lorsque le second signal se propage, une valeur de données du nœud du réseau change, et un état dans lequel la valeur de données du nœud ne change pas est défini comme un état de saturation de propagation du signal ; dans lequel (h) consiste en outre à déterminer les nœuds cibles sur la base des valeurs de données des nœuds dans l'état de saturation de propagation du signal, la valeur des données changeant dans une direction dans laquelle la valeur des données du nœud augmente proportionnellement au nombre de fois que le second signal passe par le nœud dans le processus de propagation du signal.

4. Procédé mis en œuvre par ordinateur selon la revendication 1, dans lequel, lorsqu'une catégorie de l'entité interrogée est une maladie, la troisième valeur comprend une valeur de données comparatives obtenues par comparaison des premières données biologiques d'une personne atteinte d'une maladie correspondant à l'entité interrogée avec les deuxièmes données biologiques d'une personne non atteinte d'une maladie correspondant à l'entité interrogée.

5. Procédé mis en œuvre par ordinateur selon la revendication 4, dans lequel la troisième valeur est une valeur p indiquant l'importance d'un degré d'augmentation ou de diminution de l'expression de chaque protéine ou gène d'un patient atteint d'une maladie correspondant à l'entité interrogée, ou une valeur permettant de quantifier le degré de mutation génétique d'un patient atteint d'une maladie correspondant à l'entité interrogée.

6. Procédé mis en œuvre par ordinateur selon la revendication 1, dans lequel, lorsqu'une catégorie de l'entité interrogée est un médicament, la troisième valeur comprend une valeur de changement des données biologiques apparaissant dans une entité spécifique lorsque l'entité spécifique est traitée par le médicament correspondant à l'entité interrogée.

7. Procédé mis en œuvre par ordinateur selon la revendication 6, dans lequel la troisième valeur est une valeur p indiquant l'importance d'un degré de changement d'expression pour chaque protéine ou gène lorsque l'entité spécifique est traitée par le médicament correspondant à l'entité interrogée.

8. Procédé mis en œuvre par ordinateur selon la revendication 1, dans lequel, dans (a) la courbure de chaque arête est déterminée en fonction du nombre de nœuds adjacents aux premier et second nœuds connectés l'un à l'autre par l'intermédiaire de l'arête, lorsque le nombre des troisièmes nœuds connectés au premier nœud par l'intermédiaire de l'arête N_s, le nombre de quatrièmes nœuds connectés au deuxième nœud par l'intermédiaire de l'arête est N_t et le nombre d'intersections entre les troisièmes et les quatrièmes nœuds est N_i, la courbure de chaque arête est déterminée par 4 - (N_s) - (N_t) + 3 x (N_i).

9. Procédé mis en œuvre par ordinateur selon la revendication 8, dans lequel, dans (a), un poids attribué à chaque arête est déterminé par une valeur issue d'une fonction monotone croissante dont la courbure est déterminée comme 4 - (N_s) - (N_t) + 3 x (N_i) en tant que variable.

10. Procédé mis en œuvre par ordinateur selon la revendication 9, dans lequel la fonction monotone croissante est

$$y = e^{\frac{\beta(k-\bar{k})}{sd(k)}}$$

dans laquelle y est un poids, β est un coefficient permettant de régler l'effet de la courbure sur les poids, k est la courbure de l'arête correspondante, $\bar{k}$ est la moyenne des courbures, et sd(k) correspond à l'écart-type des courbures.

11. Procédé mis en œuvre par ordinateur selon la revendication 1, dans lequel le réseau est un réseau homogène comprenant des nœuds protéiques et une relation entre les nœuds protéiques sous la forme d'une arête, l'entité interrogée est une maladie ou un médicament, et la première entité est une protéine connue pour être liée à la maladie ou au médicament.

12. Procédé selon la revendication 1, comprenant en outre, avant (a), (a0) l'extraction d'un réseau homogène comprenant des nœuds et une arête qui est une relation entre les nœuds par extraction d'un nœud d'un seul type parmi une maladie, une protéine ou un gène et un médicament d'un réseau hétérogène dans lequel une ou plusieurs maladies, protéines, gènes et médicaments sont définis comme des nœuds et une relation entre les nœuds est définie comme une arête, à l'aide du dispositif d'extraction de réseau.

13. Système (100) construit au moyen du procédé selon l'une quelconque des revendications 1 à 12.

14. Produit programme lisible par ordinateur non transitoire pour exécuter le procédé selon l'une quelconque des revendications 1 à 12.

[Fig. 1]

| NETWORK EXTRACTION DEVICE | ~110 |
| CURVATURE COMPUTATION DEVICE | ~120 |
| WEIGHT ASSIGNING DEVICE | ~130 |
| PRIOR KNOWLEDGE EXTRACTION DEVICE | ~140 |
| DATA ASSIGNING DEVICE | ~150 |
| WEIGHT CORRECTION DEVICE | ~160 |

| INPUT DEVICE | SEARCH DEVICE | OUTPUT DEVICE |
| 170 | 180 | 190 |

[Fig. 2]

```
                              ( START )
                                  │
                                  ▼
┌─────────────────────────────────────────────────────────────┐
│   COMPUTE CURVATURES OF EDGES INCLUDED IN THE NETWORK         │──S21
└─────────────────────────────────────────────────────────────┘
                                  │
                                  ▼
┌─────────────────────────────────────────────────────────────┐
│  ASSIGN A WEIGHT TO EACH OF EDGES BASED ON COMPUTED CURVATURES│──S22
└─────────────────────────────────────────────────────────────┘
                                  │
                                  ▼
┌─────────────────────────────────────────────────────────────┐
│                    INPUT QUERIED ENTITY                       │──S23
└─────────────────────────────────────────────────────────────┘
                                  │
                                  ▼
┌─────────────────────────────────────────────────────────────┐
│   ASSIGN A FIRST VALUE TO ONE OR MORE MAIN NODES CORRESPONDING│
│   TO A FIRST ENTITY KNOWN TO BE RELATED TO THE QUERIED ENTITY,│──S24
│   AND ASSIGN A SECOND VALUE TO A NODE CORRESPONDING TO A      │
│   SECOND ENTITY OTHER THAN THE FIRST ENTITY                   │
└─────────────────────────────────────────────────────────────┘
                                  │
                                  ▼
┌─────────────────────────────────────────────────────────────┐
│   PROPAGATE SIGNAL FROM THE MAIN NODE AND PROPAGATE THE FIRST │──S25
│   SIGNAL CENTERING ON AN EDGE HAVING A HIGH WEIGHT           │
└─────────────────────────────────────────────────────────────┘
                                  │
                                  ▼
┌─────────────────────────────────────────────────────────────┐
│              COMPLETE FIRST SIGNAL PROPAGATION               │──S26
└─────────────────────────────────────────────────────────────┘
                                  │
                                  ▼
┌─────────────────────────────────────────────────────────────┐
│   ASSIGN A CORRECTED WEIGHT TO EACH OF THE EDGES INCLUDED IN THE│
│   NETWORK BY INCREASING THE WEIGHT OF THE EDGE INCLUDED IN THE │──S27
│   FIRST SIGNAL PROPAGATION PATH AND DECREASING THE WEIGHT OF   │
│   THE EDGE THAT IS NOT INCLUDED IN THE FIRST SIGNAL PROPAGATION PATH│
└─────────────────────────────────────────────────────────────┘
                                  │
                                  ▼
┌─────────────────────────────────────────────────────────────┐
│   ASSIGN A THIRD VALUE IN WHICH ASSOCIATION BETWEEN THE INPUT QUERIED│
│   ENTITY AND ENTITIES CORRESPONDING TO THE PLURALITY OF NODES │──S28
│   CONSTITUTING THE NETWORK IS NUMERIZED, TO THE PLURALITY OF NODES│
└─────────────────────────────────────────────────────────────┘
                                  │
                                  ▼
┌─────────────────────────────────────────────────────────────┐
│          PROPAGATE SECOND SIGNAL BASED ON THE CORRECTED       │──S29
│              WEIGHT AND THE THIRD VALUE                       │
└─────────────────────────────────────────────────────────────┘
                                  │
                                  ▼
┌─────────────────────────────────────────────────────────────┐
│         DETERMINE ONE OR MORE OF THE NODES INCLUDED          │──S30
│   IN THE SECOND SIGNAL PROPAGATION PATH, AS TAGET NODE       │
└─────────────────────────────────────────────────────────────┘
                                  │
                                  ▼
┌─────────────────────────────────────────────────────────────┐
│        OUTPUT AN ENTITY CORRESPONDING TO THE DETERMINED      │──S31
│   TAGET NODE AS AN ENTITY RELATED TO THE QUERIED ENTITY      │
└─────────────────────────────────────────────────────────────┘
                                  │
                                  ▼
                              ( END )
```

[Fig. 3]

EDGE 2 (+1)
NODE 3 | NODE 2    EDGE 1 (−7)

NODE 1

[Fig. 4]

ASSIGN A FIRST VALUE TO ONE OR MORE MAIN NODES CORRESPONDING TO A FIRST ENTITY KNOWN TO BE RELATED TO THE QUIRED ENTITY

AFTER FIRST SIGNAL PROPAGATION STARTING FROM MAIN NODE, ASSIGN A CORRECTED WEIGHT TO EACH OF THE EDGES BASED ON THE FIRST SIGNAL PROPAGATION PATH

ASSIGN A THIRD VALUE IN WHICH ASSOCIATION BETWEEN THE INPUT QUERIED ENTITY AND ENTITIES CORRESPONDING TO THE PLURALITY OF NODES CONSTITUTING THE NETWORK IS NERMERIZED, TO THE PLURALITY OF NODES

COMPUTE CURVATURES OF EDGES AND ASSIGN A WEIGHT TO EACH OF EDGES BASED ON COMPUTED CURVATURES

AFTER SECOND SIGNAL PROPAGATION, DETERMINE ONE OR MORE TARGET NODES BASED ON THE SIGNAL PROPAGATION RESULT VALUE

[Fig. 5]

[Fig. 6]

| Network | Method | AveP | Prec@100 | Prec@200 |
|---|---|---|---|---|
| STRING | RWR | 0.085162 (---) | 0.197000 (---) | 0.159667 (---) |
| | RWR w/ GDC | 0.066500 (1.00e+00) | 0.148333 (1.00e+00) | 0.115500 (1.00e+00) |
| | mND | 0.088257 (1.81e-14) | 0.168667 (1.00e+00) | 0.170833 (7.14e-08) |
| | RWR w/ curvature | 0.084342 (1.00e+00) | 0.211333 (4.01e-08) | 0.170000 (4.61e-15) |
| | uKIN | 0.094204 (2.94e-28) | 0.223667 (6.80e-15) | 0.180000 (1.19e-17) |
| | PRESENT DISCLOSURE w/o curvature | 0.117211 (6.27e-26) | 0.304333 (1.01e-21) | 0.229667 (2.38e-22) |
| | PRESENT DISCLOSURE | **0.123157 (1.51e-24)** | **0.343667 (7.66e-24)** | **0.246333 (8.29e-24)** |
| BioGRID | RWR | 0.045023 (---) | 0.038333 (---) | 0.039667 (---) |
| | RWR w/ GDC | 0.043974 (1.00e+00) | 0.030667 (1.00e+00) | 0.035500 (1.00e+00) |
| | mND | 0.046641 (1.05e-16) | 0.030667 (1.00e+00) | 0.036000 (1.00e+00) |
| | RWR w/ curvature | 0.045154 (4.79e-02) | 0.070000 (1.37e-20) | 0.040000 (2.86e-01) |
| | uKIN | 0.047518 (1.54e-20) | 0.041667 (1.52e-02) | 0.044167 (3.42e-05) |
| | PRESENT DISCLOSURE w/o curvature | 0.053638 (7.04e-21) | 0.067000 (1.27e-08) | 0.070667 (3.05e-13) |
| | PRESENT DISCLOSURE | **0.056725 (2.13e-21)** | **0.097000 (7.42e-14)** | **0.096167 (5.94e-17)** |

[Fig. 7]

[Fig. 8]

[Fig. 9]

[Fig. 10]

**EP 4 285 367 B1**

**Patent documents cited in the description**

- KR 1020160149623

- KR 102225278

**Non-patent literature cited in the description**

- Network Enhancement: a general method to denoise weighted biological networks. ARXIV.ORG. CORNELL UNIVERSITY LIBRARY
- Curvature graph network. *International Conference on Learning Representations*

- Edge-based analysis of networks: curvatures of graphs and hypergraphs. *Theory in Biosciences*, vol. 139 (4), 337-348
- Nucleic Acids Res.. *STRING*, 2005
- Nucleic Acids Res.. *BioGRID*, 2006